## Europäisches Patentamt

## European Patent Office

## Office européen des brevets

(19)

(11) Veröffentlichungsnummer: **0 221 365**
A2

(12)

# EUROPÄISCHE PATENTANMELDUNG

(21) Anmeldenummer: 86113725.5

(22) Anmeldetag: 03.10.86

(51) Int. Cl.4: **C12P 21/00** , C07K 15/00 , C12N 5/00 , C12N 15/00 , //G01N33/569,G01N33/577,(C1-2P21/00,C12R1:91)

Der Anmelder hat eine Erklärung nach Regel 28 (4) EPÜ (Herausgabe einer Probe nur an einen Sachverständigen) eingereicht.

(30) Priorität: 19.10.85 DE 3537272

(43) Veröffentlichungstag der Anmeldung:
13.05.87 Patentblatt 87/20

(84) Benannte Vertragsstaaten:
AT BE CH DE ES FR GB GR IT LI LU NL SE

(71) Anmelder: PROGEN Biotechnik GmbH
Im Neuenheimer Feld 519
D-6900 Heidelberg(DE)

(72) Erfinder: Sethi, Kirshan Kumar, Prof. Dr. Med.
Leipzigerstrasse 12
D-6904 Eppelheim(DE)

(74) Vertreter: Hach, Hans Karl, Dr.
Tarunstrasse 23
D-6950 Mosbach-Waldstadt(DE)

(54) Monoklonaler Antikörper, der mit Legionella pneumophila reagiert.

(57) Monoklonaler Antikörper aus der Klasse IgG3, der mit den meisten Stammen der Serogruppe 1 der Legionella pneumophila reagiert, dagegen nicht reagiert mit Stämmen der Legionella pneumophila und dazu verwandten Stämmen, soweit sie nicht zur Serogruppe 1 der Legionella pneumophila gehören.

EP 0 221 365 A2

## MONOKLONALER ANTIKÖRPER, DER MIT LEGIONELLA PNEUMOPHILA REAGIERT

Die Erfindung betrifft einen monoklonalen Antikörper aus der Klasse IgG3, der mit Legionella pneumophila reagiert, ein Hybridom zur Produktion solcher Antikörper, ein Verfahren zur Herstellung dieses Hybridoms und ein Verfahren zur Herstellung der monoklonalen Antikörper mittels dieses Hybridoms.

Man kennt heute einundzwanzig nah miteinander verwandte Arten der Familie Legionella, die in Tabelle 1 aufgeführt sind. Bei Legionella pneumophila können neun Serogruppen unterschieden werden, unter denen die Vertreter der Serogruppe 1 bei menschlichen Erkrankungen am häufigsten Ursache ist.

Bei dieser Erkrankung handelt es sich um die sogenannte Legionärskrankheit, die eine akute schwere Lungenerkrankung mit hohem Fieber und radiologisch erkennbaren Veränderungen ist.

Da die Legionärskrankheit eine gefährliche Krankheit ist, ist es Aufgabe der Erfindung, ein Diagnosemittel für die Erreger dieser Krankheit zur Verfügung zu stellen.

Ein aus der EPA 0 119 893 bekannter monoklonaler Antikörper der eingangs genannten Art erfüllt diese Bedingung nicht optimal.

Die genannte Aufgabe wird gelöst durch einen Antikörper der eingangs genannten Art, der dadurch gekennzeichnet ist, daß er mit den meisten Stämmen der Serogruppe 1 der Legionella pneumophila reagiert und daß er nicht reagiert mit Stammen der Legionella pneumophila, die nicht der Serogruppe 1 angehören. Ein solcher Antikörper kann gewon nen werden mit einem Hybridom, das hinterlegt ist unter den Hinterlegungsdaten, die im Anspruch 8 angegeben sind. Dieser Antikörper reagiert mit den in Tabelle 2 angezeigten Stämmen der Serogruppe 1 der Legionella pneumophila. In dieser Tabelle sind alle bisher untersuchten Stämme aufgeführt. Bei sämtlichen untersuchten Stämmen wurde die Reaktion festgestellt.

Es ist nicht auszuschließen, daß dieser Antikörper mit allen bisher bekannten Stämmen der Serogruppe 1 reagiert, das kann aber im Augenblick noch nicht bestätigt werden, weil noch nicht alle Stämme untersucht sind. Es muß hier auch die Einschränkung gemacht werden, daß neue Stämme der Serogruppe 1 in Zukunft noch entdeckt werden, deren Reaktion mit diesem Antikörper derzeit nicht vorausgesagt werden kann.

Bemerkenswert ist allerdings, daß von allen bisher untersuchten Stämmen der Serogruppe 1 Reaktion festgestellt wurde. Das dürfte vermutlich daran liegen, daß der Antikörper optimal auf das antigene Profil der Legionella pneumophila der Serogruppe 1 ausgerichtet ist. Das ist bemerkenswert, weil der diagnostische Nachweis von Legionella pneumophila Serogruppe 1-Organismen nicht nur durch Kreuzreaktionen mit Legionella pneumophila Organismen anderer Serogruppen sondern auch durch das komplexe Antigenmuster der Organismen der Serogruppe 1 erschwert wird. Es handelt sich um zirka 85 an diesem Antigenmuster beteiligte Antigene. Diese Vielfalt wird auch durch die Angaben der Tabelle 2 deutlich.

Die besonderen Eigenschaften des Antikörpers, nämlich seine Reaktion mit den meisten Stämmen der Legionella pneumophila der Serogruppe 1 und das Ausbleiben von Reaktionen mit Stämmen, die nicht zur Serogruppe 1 gehören, macht ihn in vorteilhafter Weise zu einem selektiven und auch voll ständigen Nachweismittel für den Erreger der Legionärskrankheit.

Bemerkenswert und kennzeichnend für den monoklonalen Antikörper ist auch die Tatsache, daß er mit Agglutieren reagiert, und zwar erfolgt die agglutierende Reaktion in einer Reaktionszeit von weniger als fünf Minuten. Die agglutierende Reaktion läßt sich verhältnismäßig leicht und eindeutig beobachten und die - schnelle Reaktionszeit ist günstig für eine schnelle Diagnose.

Die Reaktion wurde geprüft durch einen Mikroagglutinationstest.

BEISPIEL 1:

Es wurde der von Farshy et al. (1979) (siehe ANHANG L.1) beschribene Mikroagglutinationstest mit geringfügigen Modifikationen angewendet:
Ein ausgewählter Legionella pneumophila Stamm wurde auf Charcoal Hefe-Extrakt (CYE) Agar nach Difco - (siehe ANHANG 1.1) angezüchtet. Die Bakterien wurden geerntet, hitzeaktiviert, dreimal gewaschen und suspendiert in PBS (pH 7.0) (siehe ANHANG 1.2) mit einer Dichte von 10000000 Bakterien per ml - (Milliliter) PBS. Anschließend wurde Carbol-Fuchsin bis zu einer Endkonzentration von 0.002 % zugegeben. Bei Zimmertemperatur wurden je 25 Microliter PAN-LPS 1 (siehe Anhang 1.9) in PBS (pH 7.0) und Bakterienproben auf Objektträgern im Gewichtsverhältnis 1 : 1 gemischt und auf Agglutination kontrolliert.

Es zeigte sich entweder nach weniger als fünf Minuten Verklumpen beziehungsweise Agglutinieren der Bakterien oder keine Reaktion.

Mit dem Mikroagglutinationstest nach Beispiel 1 wurden diejenigen Stämme der Legionella pneumophila der Serogruppe 1 die in Tabelle 2 aufgeführt sind und weitere etwa 100 Stämme getestet. Sämtliche getesteten Stämme der Legionella pneumophila der Serogruppe 1 zeigten die beschriebene Reaktion durch Agglutinieren.

Mit diesem Mikroagglutinationstest wurden auch die in Tabelle 1 aufgeführten Legionella-spezies getestet. Sie zeigten sämtlichst, mit Ausnahme der Legionella pneumophila der Serogruppe 1, keine Reaktion, auch nicht nach längerer Wartezeit von mehr als 60 Minuten.

Der monoklonale Antikörper ist geeignet zum Nachweis von Legionella pneumophila der Serogruppe 1 mit Hilfe der nachfolgend aufgeführten bekannten Test-Verfahren.

1 -Mikroagglutinationstest wie oben im Zusammenhang mit Beispiel 1 beschrieben.

2 -Immunfluoreszenztest zum direkten Nachweis der Erreger aus Untersuchungsmaterial mittels direkter oder indirekter Immunfluoreszenzmethoden, angewendet auf verschiedene Untersuchungsmaterialien.

3 -Solid-phase und/oder Sandwich-ELISA-Test zum Nachweis von Legionella pneumophila-Bakterien oder löslichem Legionella pneumophila-Antigen aus Untersuchungsmaterial.

4 -Staphylococcal Coagglutinationstest zum Nachweis von Legionella pneumophila Antigen aus Untersuchungsmaterial.

Der Solid-phase und/oder Sandwich-ELISA-Test eignet sich auch zum Nachweis des löslichen Antigen der Legionella pneumophila der Serogruppe 1. Der Antikörper eignet sich in bemerkenswerter Weise nicht nur zum direkten Nachweis der Erreger sondern auch zum Nachweis des löslichen Antigen.

Untersuchungsmaterialien nach Ziffer 1 bis 4 können zum Beispiel sein: Urin, Serum, Sputum, Pleuralexudat, Lungenpunktat und Wasserproben.

Der monoklonale Antikörper wird erhalten aus einer Maus-Maus-Hybridoma-zellkultur, die durch somatische Fusion von Milz-Zellen einer zuvor mit Legionella pneumophila der Serogruppe 1 -Stamm OLDA -(CDC) immunisierten Maus mit Maus-Myelom-Zellen und wiederholter anschließender Fusionierung und Seklektierung erhalten wurde. Dabei werden vorzugsweise Maus-Myelom-Zellen P3-X63-Ag8.653 eingesetzt. Ein solches Hybridom ist hinterlegt mit Daten wie im Anspruch 8 angegeben.

Ein Verfahren zur Herstellung dieses Hybridoms ist gekennzeichnet durch die folgenden Verfahrensschritte:

a) Animpfung einer immunsupprimierten Maus mit Legionella pneumophila der Serogruppe 1 -Stamm OLDA (CDC) durch intraperitoniale Injektion,

b) Entnahme von Milzzellen der Maus einige Tage später,

c) Fusion der Milzzellen mit Maus-Myelom-Zellen P3-X63-Ag8.653,

d) Selektion der Hybridomzellen,

e) Klonen und Kultivieren der Hybridome,

f) Testen und Selektieren der Hybridome nach Reaktionen gemäß Ansoruch 1, 2 und 3 und

g) Kultivieren des selektierten Hybridoms.


BEISPIEL 2:

Somatische Zellfusion zur Herstellung des Hybridoms

Die Milz einer immunisierten Maus wird 3 -4 Tage nach der letzten Booster-Injektion unter sterilen Bedingungen entnommen. Durch vorsichtiges Zerreiben auf einem Edelstahlsieb (Porenweite 100 Mikrometer) werden die Milzzellen aus dem Gewebeverband isoliert und in PBS = Phosphat-gepufferte Salzlösung (siehe ANHANG 1.2) aufgenommen. Die Zellen werden zweimal in DPBS (siehe ANHANG 1.3) gewaschen -(Zentrifugation bei 1000 rpm, 5 min) und anschließend in DPBS aufgenommen. In diesem Ansatz werden Milzzellen und Maus-Myelom-Zellen P3-X63-Ag8.653 im Verhältnis 2 : 1 gemischt. Durch Zugabe einer Polyäthylenglycol-Lösung = PEG 4000 (siehe ANHANG 1.4) 71%, DMSO (siehe ANHANG 1.5) 6 % in DPBS: wird die Fusion der Zellpopulation gestartet. Nach 1 min wird das PEG durch Zugabe von DPBS ausverdünnt. Die Zellsuspension wird bis zur restlosen Entfernung des PEG gewaschen und anschließend mit einer Zelldichte von ca. 1 000 000 Zellen/ml in HAT-Medium (siehe ANHANG 1.6) aufgenommen. In diesem Selektionsmedium überleben ausschließlich fusionierte Zellen, nicht jedoch die ebenfall noch in der Suspension befindlichen unfusionierten Milz-beziehungsweise Maus-Myelom-Zellen P3-X63-Ag8.653.

Ein Verfahren zur Herstellung der monoklonalen Antikörper ist gekennzeichnet durch die folgenden Verfahrensschritte:

a) Konditionieren des Peritoneums einer Maus vom Stamm BALB/C,

3

b) intraperitonales Applizieren einer Suspension von Hybridomzellen nach Anspruch 9 innerhalb eines Zeitraums von 7 -60 Tagen nach Konditionierung,

c) Extrahieren intraperitonaler, zellhaltiger Ascitesflüssigkeit durch Anstechen des Peritoneums einige Tage nach der Applizierung,

d) Abtrennen der zellulären Bestandteile von der entnommenen Ascitesflüssigkeit und

e) Gewinnen der monoklonalen Antikörper aus dem Überstand durch Reinigung.

BEISPIEL 3

VERFAHREN ZUR HERSTELLUNG DER MONOKLONALEN ANTIKÖRPER

Expansion in der Ascitesmaus (in vivo)

Mäuse vom Stamm BALB/C erhalten zur Konditionierung des Peritoneums 0,5 ml des Mineralöls Pristan (siehe ANHANG 1.7 intraperitonal).

Innerhalb eines Zeitraumes von 7 -60 Tagen wird den so vorbehandelten Tieren eine Suspension von 1 000 000 bis 10 000 000 Hybridomzellen pro Tier in PBS intraperitonial appliziert. Nach 8 -10 Tagen wird mit einer Kanüle das Peritoneum angestochen und die zellhaltige Ascitesflüssigkeit gesammelt.

Von der entnommenen Ascitesflüssigkeit werden die zellulä ren Bestandteile durch Zentrifugation abgetrennt (1000 rpm, 10 min). Der Überstand, der auch die monoklonalen Antikörper enthält, wird anschließend affinitätschromatographisch gereinigt.

Die Reinigung des Ascitesflüssigkeit erfolgt in Anlehnung an die Methode von BRUCK, die beschrieben ist unter "BRUCK et al. J. Immunol. Meth. 53. 313-319, (1982).

ANHANG

Erklärung von Abkürzungen

4

1.1 Charcoal Hefe-Extrakt (CYE) Agar nach (Difco)

| | | |
|---|---|---|
| Hefe Extrakt | 10,0 | g |
| Aktivkohle | 1,5 | g |
| L-Zystein $HCl \cdot H_2O$ | 0,4 | g |
| Lösliches Eisenpyrophosphat | 0,25 | g |
| Agar | 175 | g |
| Destilliertes Wasser | 1 | l (Liter) |

1.2 PBS = Phosphat-Puffer (Mengen in g/l)

9.6 mM, pH = 7.4

| | |
|---|---|
| NaCl | 8.0 |
| KCl | 0.2 |
| $Na_2HPO_4 \cdot 2H_2O$ | 1.44 |
| $KH_2PO_2$ | 0.2 |

1.3 DPBS = Dulbecco's PBS (Mengen in mg/l)

| | |
|---|---|
| NaCl | 8000 |
| KCl | 200 |
| $Na_2HPO_4$ | 1150 |
| $KH_2PO_2$ | 200 |
| $MgCl_2 \cdot 6H_2O$ | 100 |
| $CaCl_2$ | 100 |

1.4 PEG = Polyäthylenglykol 4000

Lieferant: Serva

1.5 DMSO = Dimethylsulfoxid

Lieferant: Merck, Darmstadt

1.6 HAT-Medium /HT-Medium

A: Aminopterin 3,82 mg/200 ml aqua. bidest.

HT: Hypoxanthin 272,20 mg

Thymidin 76,50 mg in 200 ml aqua. bidest.

HAT-Medium: 10 ml Stammlösung A + 10 ml Stammlösung HT auf 1000 ml RPMI 1640 (siehe ANHANG 1.8) (komplett mit Zusätzen)

HT-Medium: 10 ml Stammlösung HT auf 1000 ml RPMI 1640 (komplett mit Zusätzen).

1.7 Pristan = ein Mineralöl

Zusammensetzung: $C_{19}H_{40}$ MG 268,51

Das Wort "Pristan" ist ein geschütztes Warenzeichen.

Lieferant: ROTH

1.8 Medium RPMI 1640 (Mengen in mg/l)

| | |
|---|---|
| NaCl | 6000 |
| KCl | 400 |
| $Na_2HPO_4 \cdot 7H_2O$ | 1512 |
| $MgSO_4 \cdot 7H_2O$ | 100 |
| $Ca(NO_3)_2 \cdot 4H_2O$ | 100 |
| D-Glucose | 2000 |
| Phenolrot* | 5 |
| $NaHCO_3$ | 2000 |
| L-Arginin | 200 |
| L-Asparagin | 50 |
| L-Asparaginsäure | 20 |
| L-Cystin | 50 |
| L-Glutamin | 300 |
| L-Glutaminsäure | 20 |
| Glycin | 10 |
| L-Histidin | 15 |
| L-Hydroxyprolin | 20 |
| L-Isoleucin | 50 |

| | |
|---|---|
| L-Leucin | 50 |
| L-Lysin-HCl | 40 |
| L-Methionin | 15 |
| L-Phenylalanin | 15 |
| L-Prolin | 20 |
| L-Serin | 30 |
| L-Threonin | 20 |
| L-Tryptophan | 5 |
| L-Tyrosin | 20 |
| L-Valin | 20 |
| Glutathion | 1 |
| Biotin | 0,2 |
| Vitamin $B_{12}$ | 0,005 |
| D-CA-Pantothenat | 0,25 |
| Cholinchlorid | 3 |
| Folsäure | 1 |
| i-Inosit | 35 |
| Nicotinamid | 1 |
| p-Aminobenzoesäure | 1 |
| Pyridoxin . HCl | 1 |
| Riboflavin | 0,2 |
| Thiamin . HCl | 1 |

*Flüssigmedium enthält 10 mg/l Phenolrot

zusätzlich:

| | |
|---|---|
| 0.002 mol/l | l-Glutamin |
| $10^5$ U/l | Penicillin-Streptomycin |
| $2 \times 10^{-5}$ mol/l | Mercaptoäthanol |
| 10 - 15 % | FCS (fötales Kälberserum) |

1.9 PAN-LPS 1 = Breit reagierender Antikörper für Legionelle pneumophila der Serogruppe 1

LITERATUR:

L.1 = Farshy, C.E., Cruce, D.D., Klein, G.C., Wilkinson, H.W. und Feely, T.C..;
Immunglobulin specificity of the microagglutination test for the Legionnaire's disease bacterium, Ann. Inter. Med. 90 -690, 1979.

TABELLE 1

LEGIONELLA SPEZIES UND SEROGRUPPEN

---

| Name der Spezies | Sero-Gruppe | S t a m m | ATCC No. |
|---|---|---|---|
| Legionella pneumophila | 1 | Philadelphia 1 (IFA) | 33152 |
| | | Knoxville 1 (DFA) | 33153 |
| | 2 | Togus 1 | 33154 |
| | 3 | Bloomingston 2 | 33155 |
| | 4 | Los Angeles 1 | 33156 |
| | 5 | Dallas 1 | 33216 |
| | 6 | Chicago 2 | 33215 |
| | 7 | Chicago 8 | 33823 |
| | 8 | Concord 3 | 35096 |
| | 9 | IN-23-G2-C2 | 35289 |
| Legionella bozemanii | 1 | WIGA | 33217 |
| | 2 | Toronto 3 | |
| Legionella dumoffii | 1 | NY-23 | 33279 |
| Legionella gormanii | 1 | LS-13 | 33297 |
| Legionella micdadei | 1 | TATLOCK | 33218 |
| Legionella longbeachae | 1 | Long Beach 4 | 33462 |
| | 2 | Tucker 1 | 33484 |
| Legionella jordanis | 1 | BL-540 | 33623 |
| Legionella oakridgensis | 1 | Oak Ridge 10 | 33761 |
| Legionella wadsworthii | 1 | 81-716 | 33877 |
| Legionella feeleii | 1 | WO-44C-C3 | 35072 |
| | 2 | 691-WI-H | 35849 |
| Legionella sainthelensi | 1 | Mount Saint Helens 4 | 35248 |
| Legionella anisa | 1 | WA-316-C3 | 35292 |
| Leginella santicrusis | 1 | SC-63-C7 | 35301 |
| Legionella steigerwaltii | 1 | SC-18-C9 | 35302 |
| Legionella parisiensis | 1 | PF-209C-C2 | 35299 |
| Legionella spiritensis | 1 | Mount Saint Helens 9 | 35249 |
| Legionella hackeliae | 1 | Lansing 2 | 35250 |

TABELLE 1 Fortsetzung

| Name der Spezies | Sero-Gruppe | S t a m m | ATCC No. |
|---|---|---|---|
| Legionella maceachernii | 1 | PX-1-G2-E2 | 35300 |
| Legionella jamestowniensis | 1 | JA-26-G1-E2 | 35298 |
| Legionella cherrii | 1 | ORW | 35353 |
| Legionella rubrillucens | 1 | WA-270A-C2 | 35304 |
| Legionella erythra | 1 | SE-32A-C8 | 35303 |

TABELLE 2

BEKANNTE STÄMME DER LEGIONELLA PNEUMOPHILA SEROGRUPPE 1

| Stamm | Stamm | Stamm |
| --- | --- | --- |
| Derby 1 | Adelaide | New York 26 |
| San Francisco 1 | Birmingham 1 | Indianapolis 3 |
| Bloomington 1 | Allentown 1 n | West Haven 2 |
| Olda | Burlington 1 | San Francisco 9 |
| Dallas 1 | Davenport 1 | Togus 2 |
| Houston 1 | Buffalo 1 | West Haven 4 |
| Detroit 1 | New London 1 | West Haven 3 |
| Houston 3 | Kingston 1 | Bellingham 1 |
| Burlington 2 | D 5811 | Camperdown 1 |
| Portland 2 | SK 304 | München 2 |
| Long Beach 3 | Albuquerque 1 | Seed |
| Detroit 1 | West Palm Beach 1 | Notts |
| Tucson 1 | Indianapolis 1 | |
| Detroit 2 | Camblee 1 | |
| Pontiac | Stockholm 1 | |
| Orlando 1 | Concord 4 | |
| Knoxville | D 5050 | |
| Philadelphia 1 | SK 273 | |
| Los Angeles 2 | D 4999 | |
| Cambridge 3 | Berlin | |
| München 1 | Miami Beach 1 · | |
| D 5671 | RH | |
| Philadelphia 5 | Glasgow 3 | |
| SK 267 | Corby | |
| D 5006 | Zürich | |

Der eingesetzte monoklonale Antikörper wird produziert von einem Hybridom, das unter der Nr. 85100801 am 8.10.1985 bei der National Collection of Animal Cell Cultures (NCACC), Porto Down Salisbury, Wiltshire SP4 OJG, United Kingdom, hinterlegt wurde. Die hinterlegte Kultur, soll nur zugänglich gemacht werden im Sinne der Regel 28, Ziffer 4.

**Ansprüche**

1. Monoklonaler Antikörper aus der Klasse IgG3, der mit Legionella pneumophila reagiert, dadurch gekennzeichnet,
daß er reagiert mit den in Tabelle 2 angezeigten Stämmen der Serogruppe 1 der Legionella pneumophila und

daß er nicht reagiert mit den in Tabelle 1 aufgeführten zur Legionella pneumophila verwandten Legionella-Stämmen.

2. Monoklonaler Antikörper nach Anspruch 1, dadurch gekennzeichnet,

daß er mit Agglutieren reagiert.

3. Monoklonaler Antikörper nach einem der vorhergehenden Ansprüche, welche produziert worden sind vom Hybridom, das unter der Nr. 85100801 am 8.10.1985 bei der National Collection of Animal Cell Cultures (NCACC), Porton Down Salisbury, Wiltshire SP4 0JG, United Kingdom, hinterlegt wurde.

4. Hybridom hinterlegt mit den Hinterlegungsdaten wie im Anspruch 3 angegeben.

5. Hybridom, dadurch gekennzeichnet,

daß es erhalten wird durch somatische Fusion von Milz-Zellen einer zuvor mit Legionella pneumophila der Serogruppe 1 -Stamm OLDA (CDC) immunisierten Maus mit Maus-Myelom-Zellen und wiederholter anschließender Fusionierung und Selektierung und

daß es Antikörper nach einem der Ansprüche 1 bis 3 produzieren kann.

6. Monoklonaler Antikörper nach Anspruch 5, dadurch gekennzeichnet,

daß Maus-Myelom-Zellen P3-X63-Ag8.653 eingesetzt werden.

7. Verfahren zur Herstellung des Hybridom nach Anspruch 5 oder 6, gekennzeichnet durch die folgenden Verfahrensschritte:

a) Animpfung einer immunsupprimierten Maus mit Legionella pneumophila der Serogruppe 1 -Stamm OLDA (CDC) durch intraperitoniale Injektion,

b) Entnahme von Milzzellen der Maus einige Tage nach der Injektion,

c) Fusion der Milzzellen mit Maus-Myelom-Zellen P3-X63-Ag8.653,

d) Selektion der Hybridomazellen,

e) Klonen und Kultivieren der Hybridome,

f) Testen und Selektieren der Hybridome nach Reaktionen gemäß Anspruch 1, 2 und 3, und

g) Kultivieren des selektierten Hybridoms.

8. Verfahren zur Herstellen der monoklonalen Antikörper nach einem der Ansprüche 1 bis 3, gekennzeichnet durch die folgenden Verfahrensschritte:

a) Konditionieren des Peritoneums einer Maus vom Stamm BALB/C,

b) intraperitonales Applizieren einer Suspension von Hybridomzellen nach Anspruch 4 oder 5, innerhalb eines Zeitraums von 7 -60 Tagen nach Konditionierung,

c) Extrahieren intraperitonaler, zellhaltiger Ascitesflüssigkeit durch Anstechen des Peritoneums einige Tage nach der Applizierung,

d) Abtrennen der zellulären Bestandteile von der entnommenen Ascitesflüssigkeit,

e) Gewinnen der monoklonalen Antikörper aus dem Überstand durch Reinigung.